# EUROPEAN PATENT APPLICATION

(11) **EP 4 345 092 A1**
(43) Date of publication of application: **03.04.2024**
(21) Application number: 22199255.5
(22) Date of filing: 30.09.2022
(51) Int. Cl.: C07C 233/69, A61K 31/166, A61P 31/06

(54) **NITROBENZAMIDE COMPOUNDS, METHODS AND USES THEREOF**

(30) Priority: 29.09.2022 PT 2022118236
(71) Applicant: Faculdade de Farmácia da Universidade de Lisboa, 1649-003 Lisboa (PT)
(72) Inventor: VICENTE CONSTANTINO, LUIS FILIPE, 1649-003 LISBOA (PT); ALMEIDA PAIS, JOÃO PEDRO, 1700-348 LISBOA (PT); DUARTE DELGADO, TIAGO ALEXANDRE, 2705-293 COLARES (PT); ANTONIUK, OLHA, 3025-041 COIMBRA (PT); MARTINS DA SILVA, RAQUEL, 2640-734 SÃO MIGUEL DE ALCAINÇA (PT); RIBEIRO DOS SANTOS ANES, ELSA MARIA, 1649-003 LISBOA (PT); RODRIGUES PIRES, DAVID ALEXANDRE, 2790-361 QUEIJAS (PT)
(74) Representative: Patentree

(57) **Abstract**

The present disclosure relates to novel nitrobenzamide compounds and their obtention, and also their use as selective inhibitors of the enzyme DprEl. Furthermore, the present disclosure relates to the use of such compounds in medicine or veterinary, specifically, in the prevention or treatment of tuberculosis.

## Description

### TECHNICAL FIELD

The present disclosure relates to novel nitrobenzamide compounds and their obtention, and also their use as selective inhibitors of the enzyme DprE1. Furthermore, the present disclosure relates to the use of such compounds in medicine or veterinary, specifically, in the prevention or treatment of tuberculosis.

### BACKGROUND

Tuberculosis (TB) is a fatal infectious disease and is one of the top 10 causes of death worldwide, with 1.2 million deaths in 2019. The main causative agent of this disease is *Mycobacterium tuberculosis* (MTB) and although there are several drugs active against this disease, the worldwide TB burden remains. Also, there are several challenges for the current treatment, including drug resistance and toxicity. Once these occur, the treatment needs to be interrupted and changed as soon as possible[1]. Although researchers have invested a lot of time and effort in developing new drugs for TB treatment, the existing standard of care is still a challenge and a hindrance to global TB treatment. When experts encounter multi-drug resistant tuberculosis (MDR-TB), the available drugs are limited and during the last 50 years, only three drugs were discovered and approved for treatment of MDR-TB[2]. Lead compounds for new targets are still in a lead-optimization state, including decaprenylphospho-β-D-ribofuranose 2-dehydrogenase (DprE1) inhibitors[3].

DprE1 (EC 1.1.98.3) is a FAD-dependent oxidoreductase encoded by the Rv3090 gene (*dprE1*), and a component of the DprE1-DprE2 complex with the biological role of catalysing the 2-step epimerization of decaprenyl-phospho-ribose (DPR) to decaprenyl-phospho-arabinose (DPA), the sole donor of Ara*f* residues in the MTB cell wall synthesis, making it essential for the proliferation of Mtb. Hence, DprE1 revealed itself to be an attractive target for the development of new drugs[4].

In 2007 it was patented the first class of inhibitors of DprE1, the nitrobenzothiazinones (BTZ) [5], most notably the compound BTZ-043, although it was only in 2009 that the mechanism of action of this drug was associated with that enzyme [6]. Soon after, in an independent high throughput screening study, another class of drugs was found - the DNBs[7].

Inhibitors with nitro aromatic moieties are largely described as covalent inhibitors, and all share the mode of action. The active site of DprE1 is able to reduce the nitro group to a nitroso group, that then reacts with Cys387, forming a covalent adduct with it. This type of inhibition can also be designated as suicide inhibitors[8].

Two noteworthy aspects from this mode of action are that i) it requires the FAD to be reduced before the action of the inhibitors, FADH₂ enabling the reduction of the nitro group; and ii) since the residue Cys387 is essential, its point mutation leads to resistance to these inhibitors, as has been widely described.[9]

In addition to the most well-known class of inhibitors - the BTZ family, a number of DNBs have also been identified as interesting dprE1 inhibitors. - DNBs can be seen as a simplification of the BTZs, as taking the benzothiazinone scaffold and opening the heterocycle ring at the sulphur atom, leading to a structure that maintains the same essential features. [10].

The DNB family of compounds has seen little development since discovery, probably due to the emergence of other new families with large structural differences and very good activity[10-12], hence, DNBs can be considered underexplored.

A general structure for DNBs can be broken down into three main sections: the core moiety containing a nitro group, a terminal group, mainly a substituted aromatic moiety and the linker portion that connects the two. This linker region can be as small as a single CH2 residue or as complex as several ring-containing moieties, most regularly, piperazine, aromatic or combinations of them[7,11].

The development of novel compounds with improved activity as DprE1 remains an important topic and would constitute an important progress in the fight against tuberculosis infections.

These facts are disclosed in order to illustrate the technical problem addressed by the present disclosure.

### GENERAL DESCRIPTION

The present disclosure relates to the development of a library of compounds, composed of novel nitrobenzamides comprising more flexible linkers.

Based on the International Union of Pure and Applied Chemistry (IUPAC) definitions, an alkyl group is defined as a univalent group derived from alkanes by removal of a hydrogen atom from any carbon atom -CₙH₂ₙ₊₁. The groups derived by removal of a hydrogen atom from a terminal carbon atom of unbranched alkanes form a subclass of normal alkyl (n-alkyl) groups H (CH₂)ₙ. The groups RCH₂, R₂CH (R ≠ H), and R₃C (R ≠ H) are primary, secondary and tertiary alkyl groups, respectively.

"Alkyl" includes "lower alkyl" and extends to cover carbon fragments having up to 30 carbon atoms. Examples of alkyl groups include octyl, nonyl, norbornyl, undecyl, dodecyl, tridecyl, tetradecyl, pentadecyl, eicosyl, 3,7-diethyl-2,2-dimethyl-4 -propylnonyl, 2-(cyclododecyl)ethyl, adamantyl, and the like.

"Lower alkyl" means alkyl groups of from 1 to 7 carbon atoms. Examples of lower alkyl groups include methyl, ethyl, propyl, isopropyl, butyl, sec- and tert-butyl, pentyl, hexyl, heptyl, cyclopropyl, cyclobutyl, cyclopentyl, cyclohexyl, cycloheptyl, 2-methylcyclopropyl, cyclopropylmethyl, and the like.

Alkyl, alkenyl and alkynyl chain comprises branched and unbranched chains, substituted or non-substituted. Preferably, in the present disclosure, alkyl, alkenyl and alkynyl chain relates to unbranched and non-substituted chains.

An as example, in the present disclosure, a C₄ chain represents a branched or unbranched alkyl, alkenyl or alkynyl chain with 4 carbons.

An aspect of the present disclosure relates to a benzamide compound of formula I or a pharmaceutically acceptable salt, or ester or solvate thereof 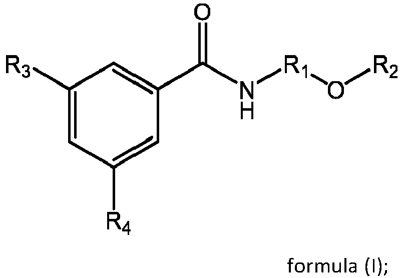
wherein R₁, R₂, R₃ and R₄ are independently selected from each other;
R₁ is an alkyl chain, an alkenyl chain or an alkynyl chain with at least 2 carbons;
R₂ is selected from H or a substituted or unsubstituted aryl;
R₃ and R₄ are selected from NO₂, CF₃, OCF₃, F, Cl, Br, I, H, CN, OCH₃, CH₃;
provided that if R₁ is a C₂ chain then R₂ is not: a fluor-substituted phenyl, a chloro-substituted phenyl, a phenyl, a methoxyphenyl, a hydroxyphenyl, a trifluoromethoxyphenyl, a benzoate, or an amino-phenyl;
provided that if R₁ is a C₃ chain then R₂ is not 4-methoxyphenyl.

In an embodiment, R₁ is an unsubstituted alkyl chain.

In an embodiment, R₃ and R₄ are NO₂.

In an embodiment, R₃ is NO₂ and R₄ is CF₃.

In an embodiment, if R₃ and R₄ are NO₂ and R₁ is a C₂ chain then R₂ is selected from: H; biphenyl; 4-bromophenyl; 4-benzylphenyl; 4-trifluoromethylphenyl.

In an embodiment, R₁ is an alkyl chain, an alkenyl chain or an alkynyl chain with at least 3 carbons; preferably an alkyl chain with at least 3 carbons.

In an embodiment, R₁ is an alkyl chain, an alkenyl chain or an alkynyl chain with at least 4 carbons; preferably an alkyl chain with at least 4 carbons.

In an embodiment, the R₁ chain is an alkyl chain, an alkenyl chain or an alkynyl chain ranging from C₃ - C₃₀; preferably C₄-C₃₀; more preferably C₅-C₃₀.

In an embodiment, the R₁ chain is an alkyl chain, an alkenyl chain or an alkynyl chain ranging from C₃-C₁₁; preferably C₄-C₁₁; more preferably C₅-C₁₁.

In an embodiment, R₁ is a C₃ chain; C₄ chain; C₅ chain; C₆ chain; C₇ chain; C₈ chain; C₉ chain; C₁₀ chain; or C₁₁ chain.

In an embodiment, R₂ is a 4-substituted phenyl selected from: 4-fluorophenyl; 4-biphenyl; 4-(trifluoromethyl)phenyl; 4-(trifluoromethoxy)phenyl; 4-nitrophenyl; 4-clorophenyl;4-benzylphenyl; 4-bromophenyl; 4-methoxyphenyl; 4-benzylphenyl; 4-biphenyl.

In an embodiment, R₁ is a C₃ chain and R₂ is selected from: biphenyl; 4-(trifluoromethoxy)phenyl; 4-fluorophenyl; 4-(trifluoromethyl)phenyl; phenyl.

In an embodiment, R₁ is a C₅ chain and R₂ is selected from: biphenyl; 4-methoxyphenyl; 4-(trifluoromethoxy)phenyl; 4-nitrophenyl; 4-fluorophenyl; 4-clorophenyl; 4-(trifluoromethyl)phenyl; 4-benzylphenyl; 4-bromophenyl; phenyl.

In an embodiment, R₁ is a C₉ chain and R₂ is 4-methoxyphenyl or phenyl.

In an embodiment, R₁ is a C₁₁ chain and R₂ is 4-methoxyphenyl or phenyl.

In an embodiment, R₁ is a C₉ chain and R₂ is (C₈H₁₆)CH₂OH or H.

In an embodiment, R₂ is H and R₁ is selected from: C₅ chain; C₃ chain; C₂ chain; and C₁₁ chain.

In an embodiment, the compound is 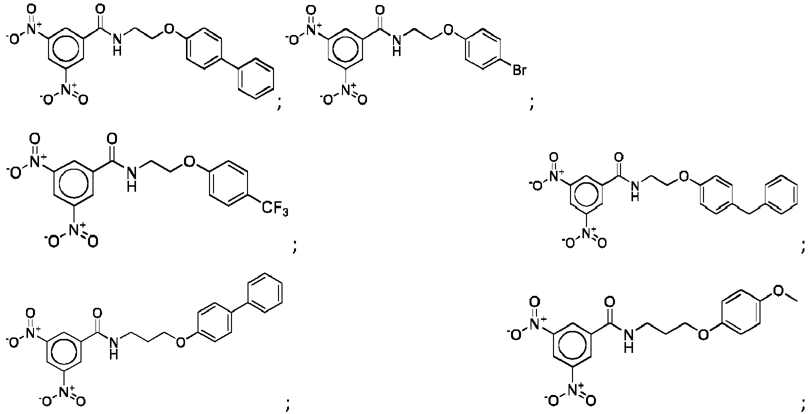 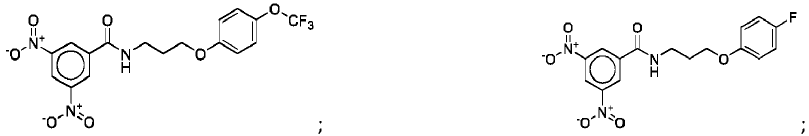 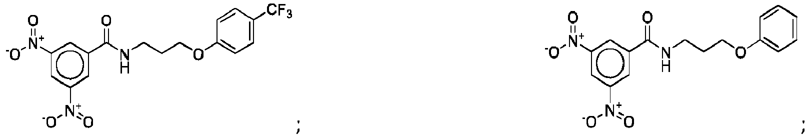 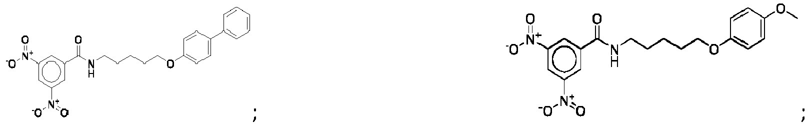 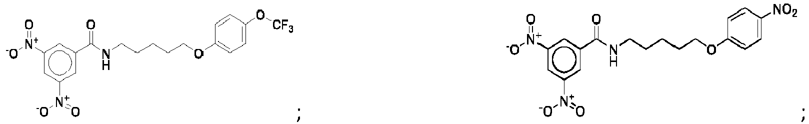 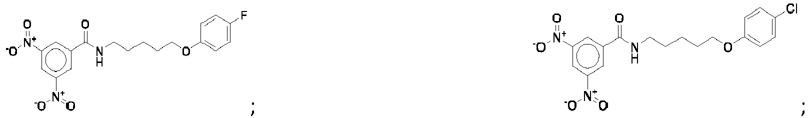 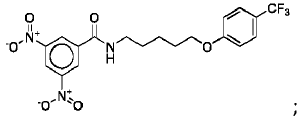 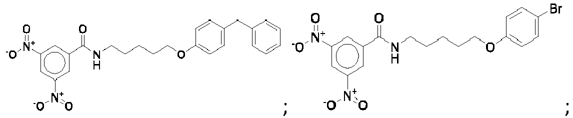 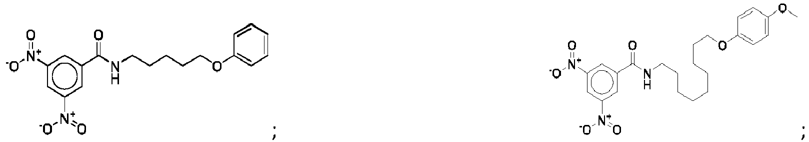 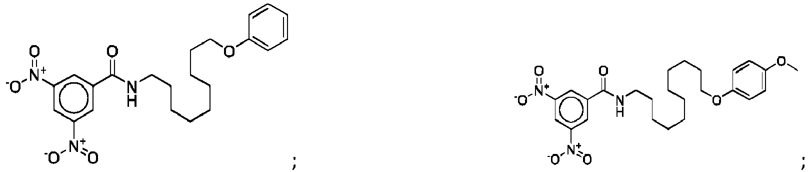 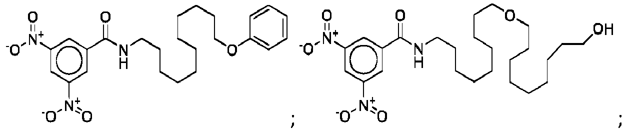 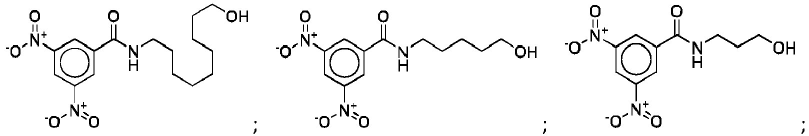 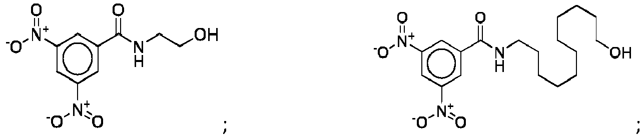 or

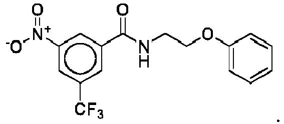

Another aspect pf the present disclosure relates to a benzamide compound of formula II or a pharmaceutically acceptable salt, or ester or solvate thereof 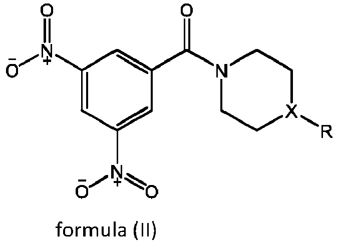
wherein R and X are independently selected from each other;
X is selected from N; O; CH;
R is selected from Me2, benzyl; OPh-(4-OCH₃); OH; H;
provided that if X is O than R is absent.

In an embodiment, X is N and R is selected from: Me2; benzyl; H.

In an embodiment, X is CH and R is selected from: benzyl group; OPh-(4-OCH₃); OH; H.

In an embodiment, X is O and R is absent.

In an embodiment, the compound is: 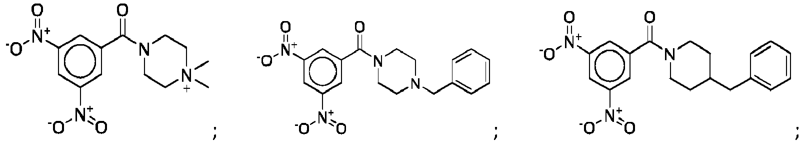 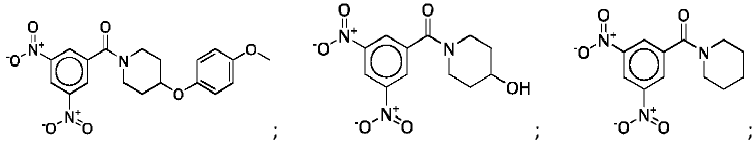 or

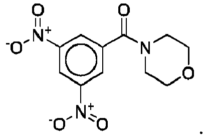

Another aspect of the present disclosure relates to a benzamide compound according to the formula:

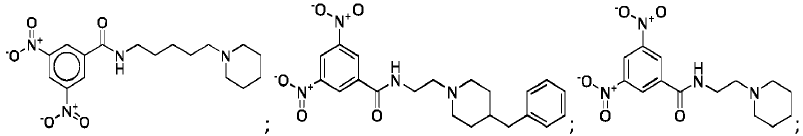

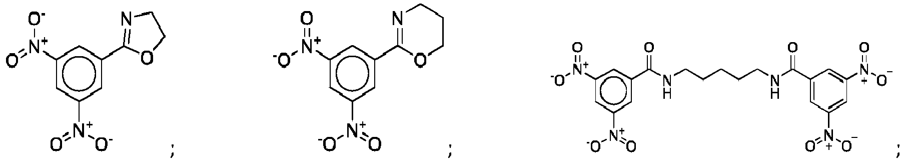

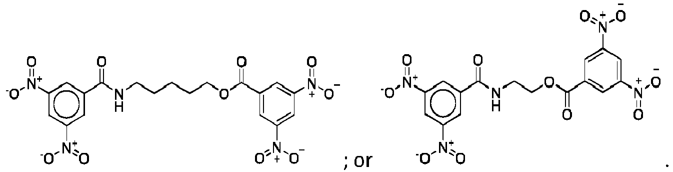

Another aspect of the present disclosure relates to the use of the compounds herein described in medicine or veterinary.

In an embodiment, the compounds may be used in any condition susceptible of being improved or prevented by selective inhibition of the enzyme DprE1.

In an embodiment, the compounds may be used in the treatment or prevention of a mycobacterial infection.

In an embodiment, the mycobacterial infection is caused by a non-tuberculous mycobacteria (NTM) selected from the group consisting of: *Mycobacterium avium complex (MAC), Mycobacterium smegmatis, Mycobacterium gordonae, Mycobacterium kansasii, Mycobacterium terrae, Mycobacterium scrofulaceum, Mycobacterium vaccae, Mycobacterium marinum, Mycobacterium lentiflavum, Mycobacterium fortuitum, Mycobacterium chelonae, Mycobacterium abscessus, Mycobacterium intracellulare and Mycobacterium avium.*

In an embodiment, the compounds may be used in the treatment or prevention of tuberculosis.

In an embodiment, tuberculosis is caused by an organism from the *Mycobacterium Tuberculosis* Complex; preferably *Mycobacterium bovis or Mycobacterium tuberculosis.*

In an embodiment, the tuberculosis disease is selected from the group consisting of primary tuberculosis disease, post-primary pleuro-pulmonary tuberculosis disease, post-primary extra-pulmonary tuberculosis disease involving at least one organ or system of a mammal.

In an embodiment, the compounds may be used in combination with at least one anti-HIV agent, wherein the anti-HIV agent is selected from a HIV protease inhibitor, a HIV nucleoside reverse transcriptase inhibitor, a HIV non-nucleoside reverse transcriptase inhibitor, or a HIV integrase inhibitor.

In an embodiment, the compounds may be used in combination with at least a second tuberculosis drug, wherein the further tuberculosis drug is selected from the group of isoniazid, rifamycin and derivatives, pyrazinamide, ethambutol, cycloserine, ethionamide, streptomycin, amikacin, kanamycin, rifampin (rifampicin), aminoglycosides, capreomycin, p-aminosalicyclic acid, fluoroquinolones such as levofloxacin, ecarbonate or gatifloxacin, or mixtures thereof.Another aspect of the present disclosure relates to the use of the compounds herein disclosed as a DprE1 inhibitor.

Another aspect of the present disclosure relates to a pharmaceutical composition comprising a compound herein described and a pharmaceutical acceptable excipient.

In an embodiment, the pharmaceutical composition comprises a therapeutically effective amount of a compound herein described or a pharmaceutically acceptable salt or solvate thereof; and (ii) a pharmaceutically acceptable excipient.

It was surprisingly found that the presence of a flexible linker between the nitrobenzamide and the aromatic ring as observed in formula (I) allow to obtain compounds with improved activity DprE1 inhibitors. This finding was not expected considering the information available in the prior art, where is claimed that increasing the alkyl linker separating the amide from the terminal aromatic ring leads to lower activities [12].

The antimycobacterial activity of the compounds was assessed against the H37Rᵥ strain of Mtb.

### DETAILED DESCRIPTION

The present disclosure relates to novel nitrobenzamide compounds and their obtention, and also their use as selective inhibitors of the enzyme DprE1. Furthermore, the present disclosure relates to the use of such compounds in medicine or veterinary, specifically, in the prevention or treatment of tuberculosis.

### Results

### Synthesis of alkyl DNBs

Multiple approaches were used in order to obtain the desired derivatives, since linkers with n= 2 carbon derivatives could not be obtained via the same synthetic pathway than when the number of Carbons is 3, 5, 9 or 11. The optimized methodologies employed are described in the method section. **Table 1** provides a comprehensive list of the derivatives synthesized, including intermediaries and secondary products, and their antimycobacterial activities.

**Table 1. List of the derivatives synthesized.**

| A | | | B | | | C | | |
|---|---|---|---|---|---|---|---|---|
| 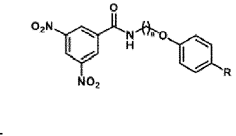 | | | 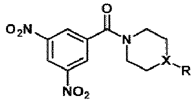 | | | 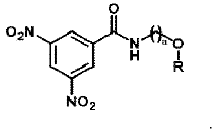 | | |
| | | | | | | | | |

| **#** | **Compound** | **Scaffold** | **R** | **n** | **X** | **MIC (µg/mL)** | **MIC (nM)** | **MBC (µg/mL)** |
|---|---|---|---|---|---|---|---|---|
| 1 | DNC2OphPh | A | Ph | 2 | - | 0.063 | 154.65 | - |
| 2 | DNC2OphOMe | A | Ome | 2 | - | 0.016 | 44.28 | 0.031 |
| 3 | DNC2Oph | A | H | 2 | - | 0.031 | 93.58 | 0.063 |
| 4 | DNC2OphF | A | F | 2 | - | 0.063 | 180.37 | - |
| 5 | DNC2OphBr | A | Br | 2 | - | 0.063 | 153.59 | - |
| 6 | DNC2OphCF3 | A | CF₃ | 2 | - | 0.125 | 313.06 | - |
| 7 | DNC2OphBn | A | Bn | 2 | - | 0.063 | 149.50 | - |
| 8 | DNC3OphPh | A | Ph | 3 | - | 0.063 | 149.50 | 0.125 |
| 9 | DNC3OphOMe | A | Ome | 3 | - | 0.063 | 167.85 | 0.125 |
| 10 | DNC3OPhOCF3 | A | OCF₃ | 3 | - | 0.063 | 146.75 | 0.25 |
| 11 | DNC3OphF | A | F | 3 | - | 0.063 | 173.41 | 0.125 |
| 12 | DNC3OphCF3 | A | CF₃ | 3 | - | 0.125 | 302.44 | - |
| 13 | DNC3Oph | A | H | 3 | - | 0.063 | 182.44 | 0.125 |
| 14 | DNC5OphPh | A | Ph | 5 | - | 0.063 | 140.17 | 0.125 |
| 15 | DNC5OphOMe | A | Ome | 5 | - | 0.063 | 156.18 | 0.125 |
| 16 | DNC5OPhOCF3 | A | OCF₃ | 5 | - | 0.031 | 67.78 | 0.063 |
| 17 | DNC5OphNO2 | A | NO₂ | 5 | - | 0.031 | 74.10 | 0.125 |
| 18 | DNC5OphF | A | F | 5 | - | 0.031 | 79.21 | 0.063 |
| 19 | DNC5OphCl | A | Cl | 5 | - | 0.016 | 39.23 | 0.063 |
| 20 | DNC5OphCF3 | A | CF₃ | 5 | - | 0.031 | 70.24 | 0.063 |
| 21 | DNC5OphBn | A | Bn | 5 | - | 0.063 | 135.93 | 0.25 |
| 22 | DNC5OphBr | A | Br | 5 | - | 0.031 | 68.54 | 0.063 |
| 23 | DNC5Oph | A | H | 5 | - | 0.031 | 83.03 | 0.063 |
| 24 | DNC9OphOMe | A | Ome | 9 | - | 0.016 | 34.82 | 0.031 |
| 26 | DNC11OphOMe | A | Ome | 11 | - | 0.063 | 129.22 | 0.125 |
| 28 | DnpzMe2 | B | Me₂ | - | N | >16 | - | >16 |
| 29 | DNpzBn | B | Bn | - | N | 2 | 5400.08 | 4 |
| 31 | DnpipBn | B | Bn | - | CH | 0.5 | 1353.63 | 2 |
| 32 | Dnpip4OPhOMe | B | OPh-(4-Ome) | - | CH | 2 | 4982.81 | - |
| 33 | Dnpip4OH | B | OH | - | CH | >16 | - | 16 |
| 34 | Dnpip | B | H | - | CH | >16 | - | 16 |
| 35 | DNMo | B | - | - | O | >16 | - | >16 |
| 36 | DNC9C9 | C | (C₈H₁₆)CH₂OH | - | - | 0.063 | 127.11 | - |
| 37 | DNC9 | C | H | - | - | 0.25 | 707.46 | - |
| 38 | DNC5 | C | H | - | - | 2 | 6727.96 | - |
| 39 | DNC3 | C | H | - | - | 4 | 14858.12 | - |
| 40 | DNC2 | C | H | - | - | 16 | 62699.37 | - |
| 41 | DNC11 | C | H | - | - | 0.125 | 327.71 | 0.25 |
| 42 | 3N5CFC2Oph | 1 | H | - | - | 0.25 | 671.39 | - |
| 43 | DNNPhOMe | 1 | - | - | - | >16 | >50000 | - |
| 44 | DNC5pip | 1 | - | 5 | - | 16 | 43907.56 | >16 |
| 47 | DNC2Cyclized | 1 | - | 2 | - | 2 | 8432.73 | 4 |
| 48 | DNC3Cyclized | 1 | - | 3 | - | 16 | 63694.77 | >16 |
| 49 | DN(NC5N)DN | 1 | - | 5 | - | 0.25 | 509.80 | - |
| 50 | DNC5DN | 1 | - | 5 | - | 0.063 | 128.21 | 0.5 |
| 51 | DNC2DN | 1 | - | 2 | - | >16 | >35600 | - |

| | | | | | | | | |
|---|---|---|---|---|---|---|---|---|
| MIC: Minimal inhibitory concentration MBC: Minimal bactericidal concentration ¹ The chemical structure of these compounds are depicted in **Table 2.** | | | | | | | | |

**Table 2. Compounds synthetized and the respective chemical structure.**

| **Compound** | **Family** | **#** | |
|---|---|---|---|
| DNC2OphPh | A | 1 | 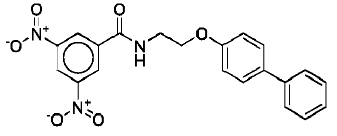 |
| DNC2OphBr | A | 5 | 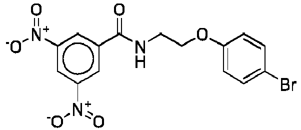 |
| DNC2OphCF3 | A | 6 | 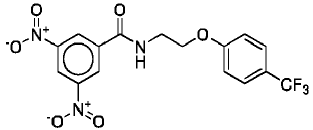 |
| DNC2OphBn | A | 7 | 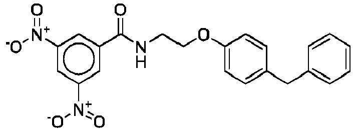 |
| DNC3OphPh | A | 8 | 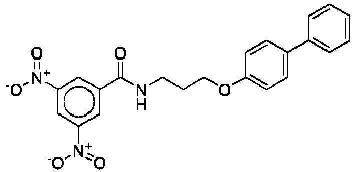 |
| DNC3OphOMe | A | 9 | 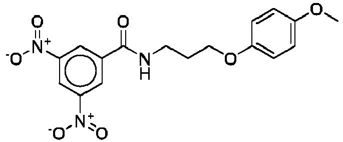 |
| DNC3OPhOCF3 | A | 10 | 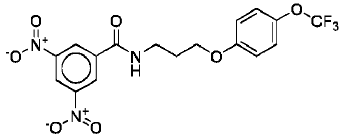 |
| DNC3OphF | A | 11 | 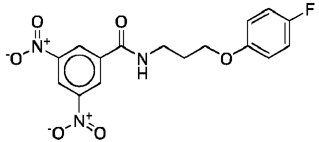 |
| DNC3OphCF3 | A | 12 | 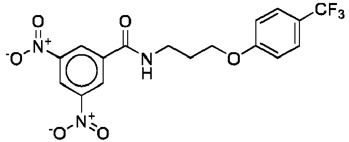 |
| DNC3Oph | A | 13 | 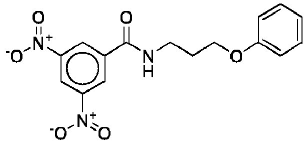 |
| DNC5OphPh | A | 14 | 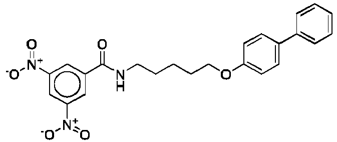 |
| DNC50phOMe | A | 15 | 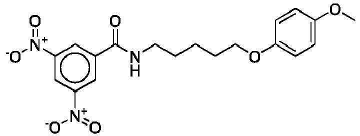 |
| DNC5OPhOCF3 | A | 16 | 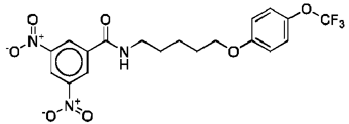 |
| DNC5OphNO2 | A | 17 | 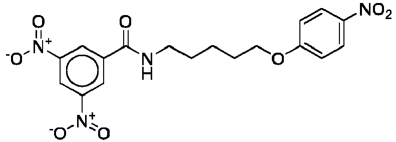 |
| DNC5OphF | A | 18 | 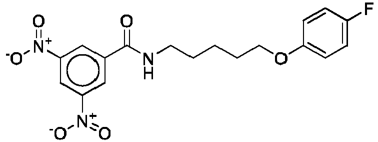 |
| DNC5OphCl | A | 19 | 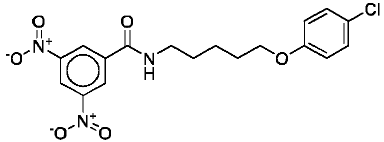 |
| DNC5OphCF3 | A | 20 | 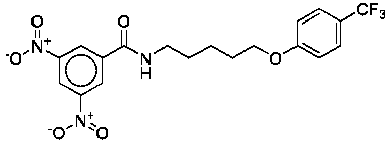 |
| DNC5OphBn | A | 21 | 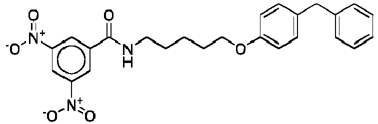 |
| DNC5OphBr | A | 22 | 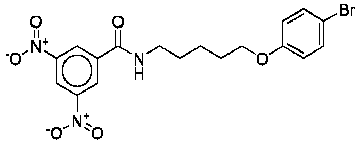 |
| DNC5Oph | A | 23 | 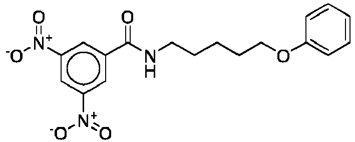 |
| DNC9OphOMe | A | 24 | 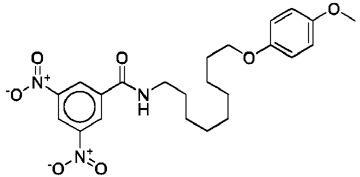 |
| DNC9Oph | A | 25 | 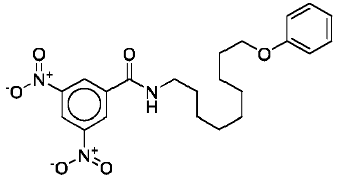 |
| DNC11OphOMe | A | 26 | 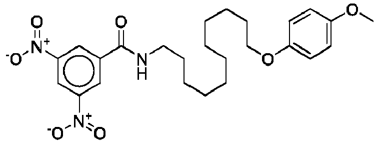 |
| DNC11Oph | A | 27 | 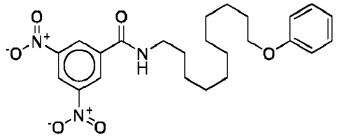 |
| DnpzMe2 | B | 28 | 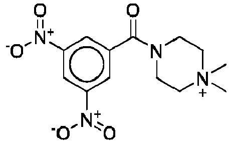 |
| DNpzBn | B | 29 | 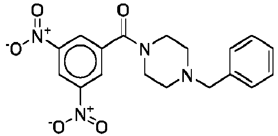 |
| DnpipBn | B | 31 | 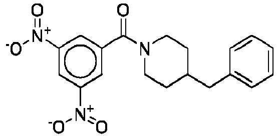 |
| Dnpip4OphOMe | B | 32 | 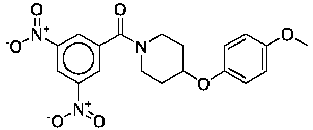 |
| Dnpip4OH | B | 33 | 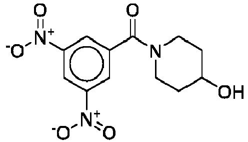 |
| Dnpip | B | 34 | 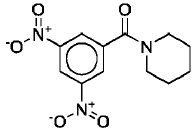 |
| DNMo | B | 35 | 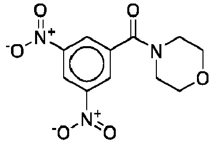 |
| DNC9C9 | C | 36 | 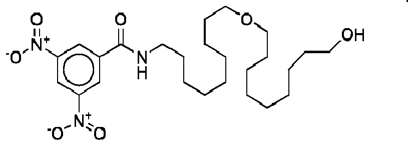 |
| DNC9 | C | 37 | 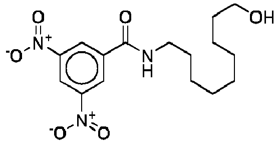 |
| DNC5 | C | 38 | 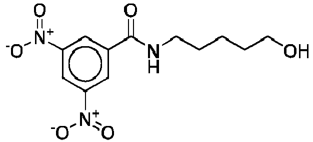 |
| DNC3 | C | 39 | 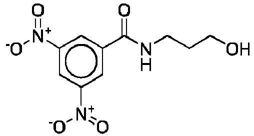 |
| DNC2 | C | 40 | 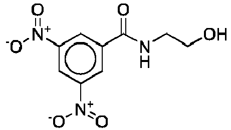 |
| DNC11 | C | 41 | 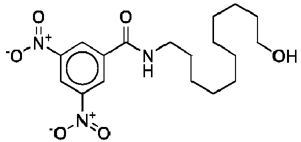 |
| 3N5CFC2Oph | n/a | 42 | 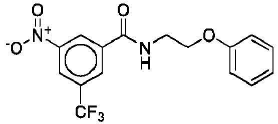 |
| DNNPhOMe | n/a | 43 | 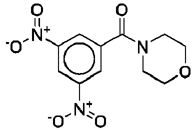 |
| DNC5pip | n/a | 44 | 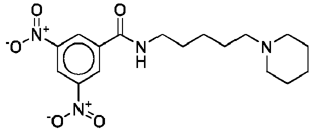 |
| DNC2pipBn | n/a | 45 | 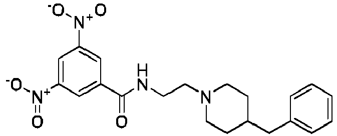 |
| DNC2pip | n/a | 46 | 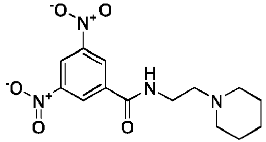 |
| DNC2Cyclized | n/a | 47 | 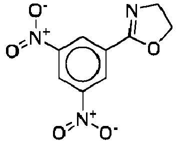 |
| DNC3Cyclized | n/a | 48 | 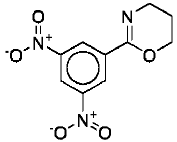 |
| DN(NC5N)DN | n/a | 49 | 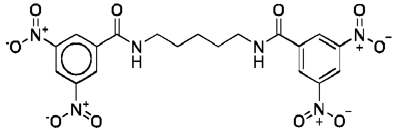 |
| DNC5DN | n/a | 50 | 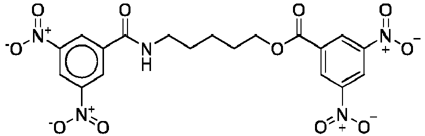 |
| DNC2DN | n/a | 51 | 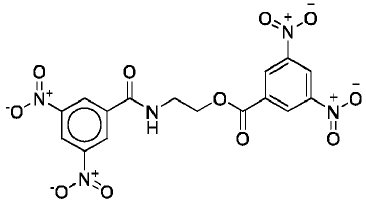 |

Analysing the data for some of the derivatives synthesized, the antitubercular activity observed for compounds 36 to 41 shows a clear trend of increasing activity with the chain length.

By comparing compounds varying only the terminal groups, for example, compounds 14- 23, no clear trend could be observed. This could indicate that the region in the target where these groups stay is large enough to encompass all the widely diverse set of terminal groups, presenting a low impact in the biological activity.

### Pharmaceutical formulations containing the compounds of the present invention

In one realization to obtain pharmaceutical formulations of the compounds amenable to being administered enterally or parenterally, liposomes containing compounds 24 and 36 were prepared according to the method described later. Briefly, Dimyristoylphosphatidylcholine (DMPC), dimyristoylphosphatidylglycerol (DMPG) and the test compound were weighed to be in the desired molar ratio, transferred to a round bottom flask, dissolved in dichloromethane, and the organic solvent was evaporated in an evaporator to form a lipid film. 1 ml of isotonic phosphate buffer pH 7.4 (PBS) was added to the lipid film and the flask was stirred to hydrate the film completely, after which the mixture was placed in an ultrasonic bath for 3 periods of 2 minutes, interspersed with 2 minutes resting time.

The following table show encapsulation efficiency (EE) results obtained with various formulations of different compounds.

| **Compound** | **Lípidos usados** | **EE** |
|---|---|---|
| 24 | DMPC:DMPG 9:1 | 80% |
| 36 | DMPC:DMPG 9:1 | 86% |

All formulations obtained showed high EE. The formulations if obtained under sterile conditions can be administered enterically. Although unusual the liposomal formulations can also be used to solubilize the drug to obtain an oral formulation or one that can be administered in aerosol form. One skilled in the art can easily obtain other pharmaceutical formulations with the compounds of the present invention.

### Materials and Methods

### Synthesis

**3,5-dinitro benzoyl chloride synthesis:** A solution of 3,5-dinitrobenzoic acid in thionyl chloride (2.5 mL per mmol of acid) was refluxed overnight, leading to the formation of the desired acyl chloride. The excess thionyl chloride is then removed by under reduced pressure and the product is used without further purification.

**Aminoalkanol synthesis (method A** - **Delèpine):** 2 mmol of 9-bromononan-1-ol or 11-bromononan-1-ol and 2.2 mmol of Hexamethylenetetramine (HMTA) were added to a round bottom flask and dissolved in 15 mL of EtOAc. Then the reaction was stirred and heated to 60°C for 6 days. Then, the solid was filtered off and dissolved in 2.5 mL of MeOH and 1 mL of HCl solution (33%). The solution was stirred at reflux for 1,5h. Afterwards, the solution was basified with K₂CO₃ until the pH = 13 and stirred for 4h. Finally, the solvent was evaporated, and the mixture was used without further purification.

**Aminoalkanol synthesis (method B** - **Staudinger):** 2 mmol of 9-bromononan-1-ol or 11-bromononan-1-ol and 3 mmol of NaN₃ were dissolved in 10mL of ACN. The mixture was heated to 60°C and stirred for 4 days. Afterwards the solvent was evaporated, the residue dissolved in EtOAc and washed with distilled water. The organic phase was dried with anhydrous Na₂SO₄ and evaporated under reduced pressure. The product was then purified by column chromatography, with yields of 74% for 9-azidononan-1-ol and 85% for 11-azidoundecan-1-ol.
The products were Then dissolved in 6 mL of THF, and 2.2 equivalents of PPh3 were added, the reaction was placed under N₂ atmosphere and it was stirred for 24h. Then, 0.1mL of water were added, and the reaction was stirred for 24h. After, the solvent was evaporated, and the mixture was used without further purification.

**Synthesis of hydroxyalkyl 3,5-dinitrobenzamides (nucleophilic addition/elimination):** 2 mmol of 3,5-dinitrobenzoyl chloride previously prepared were dissolved in 5mL of EtOAc. A second solution of 3 equivalents of the desired aminoalkanol and 1.5 equivalents of K₂CO₃ dissolved in 30 mL of EtOAc was prepared and stirred for 15 min. To it, the acyl chloride solution was added dropwise and stirred until the reaction was complete, monitored by TLC. Then, the reaction was washed with distilled water, the organic phase was dried with anhydrous Na₂SO₄, and evaporated under reduced pressure. The products were purified by column chromatography.

### N-(2-hydroxyethyl)-3,5-dinitrobenzamide (DNC2)

Yellow solid. Yield=40%. ¹H NMR (400 MHz, CDCl3): δ 8.98 (m; 3H; H-2, H-4, H-12), 3.61 (t; J = 5.2 Hz; 2H, H-10), 3.42 (t; J = 5.2 Hz; 2H; H-9). ¹³C NMR (101 MHz, CDCl3): δ 163.71 (C-6), 148.43, 137.71 (C-1, C-3, C-5), 127.55 (C-4, C-12), 120.74 (C-2), 60.38 (C-10), 42.75 (C-9).

### N-(3-hydroxypropyl)-3,5-dinitrobenzamide (DNC3)

White solid. Yield=50%. ¹H NMR (400 MHz, DMSO-D6): δ 9.16 (t; *J* = 5.4 Hz; 1H; H-8), 9.04 (m; 2H; H-13, H-4), 8.94 (t; *J* = 2.2 Hz; 1H; H-2), 4.51 (t; *J* = 5.1 Hz; 1H; H-12), 3.48 (q; *J* = 6.0 Hz; 2H; H-11), 3.38 (q, *J* = 6.7 Hz, 2H; H-9), 1.72 (p, *J* = 6.6 Hz, 2H; H-10). ¹³C NMR (101 MHz, DMSO-D6): δ 162.00 (C-6), 148.19, 137.11 (C-1, C-3, C-5), 127.46 (C-4, C-13), 120.73 (C-2), 58.47 (C-11), 37.09 (C-9), 32.08 (C-10).

### N-(5-hydroxypentyl)-3,5-dinitrobenzamide (DNC5)

White-yellowish solid. Yield=78%. ¹H NMR (400 MHz, DMSO-D6): δ 9.17 (t; J = 5.8 Hz; 1H; H-8), 9.06 (d; J = 2.1 Hz; 2H; H-4, H-15), 8.95 (t; J = 2.1 Hz; 1H; H-2), 4.39 (t; J = 5.1 Hz; 1H; H-14), 3.40 (q; J = 6.0 Hz; 2H; H-13), 3.32 (t; J = 6.4 Hz; 2H; H-9), 1.57 (p; J = 7.2 Hz; 2H; H-12), 1.46 (p; J = 6.6 Hz; 2H; H-10), 1.35 (tt; J = 8.6, 5.2 Hz; 2H; H- 11). ¹³C NMR (101 MHz, DMSO-D6): δ 161.92 (C-6), 148,22, 137.10 (C-1, C-3, C-5), 127.47 (C-4, C-15), 120.76 (C-2), 60.62 (C-13), 39,79 (C-9), 32.23 (C-12), 28.71 (C-10), 23.07 (C-11).

### N-(9-hydroxynonyl)-3,5-dinitrobenzamide (DNC9)

White-yellowish solid. Yield=50%. ¹H NMR (400 MHz, CDCl3): δ 9.05 (m; 1H; H-18), 8.99 (m; 2H; H-1, H-16), 3.49 (td; J = 6.7; 2.5 Hz; 2H; H-3), 3.36 (t; J = 7.2 Hz; 2H; H-11), 1.56 (p; J = 7.3 Hz; 2H; H-10), 1.45 (t; J = 6.8 Hz; 2H; H-4), 1.34-1.18 (m; 10H; H-5, H-6, H-7, H-8, H-9). ¹³C NMR (101 MHz, CDCl3): δ 163.23 (C-14), 148.49, 138.22 (C-15, C-17, C-19), 127.62 (C-1, C-16), 120.70 (C-18), 62.38 (C-3), 40.57 (C-11), 32.39 (C-4), 29.35-25.62 (C-5, C-6, C-7, C-8, C-9, C-10).

### N-(9-(nonyloxy)nonyl)-3,5-dinitrobenzamide (DNC9C9)

White-yellowish solid. ¹H NMR (400 MHz, CDCl3): δ 9.13 (t; J = 2.3 Hz; 1H; H-1), 8.99 (d; J = 2.2 Hz; 2H; H-3, H-28), 7.10 (t; J = 5.7 Hz; 1H; H-25), 3.62 (t; J = 6.7 Hz; 2H; H-6), 3.49 (q; J = 6.8 Hz; 2H; H-24), 3.38 (t; J = 6.6 Hz; 4H; H-14, H-16), 1.64 (p; J = 7.3 Hz; 2H; H-23), 1.54 (td; J = 7.0; 3.8 Hz; 6H; H-7, H-13, H-17), 1.42 - 1.21 (m; 20H; H-8, H-9, H-10, H-11, H-12, H-18 H-19, H-20, H-21, H-22). ¹³C NMR (101 MHz, CDCl3): δ 162.89 (C-26), 148.68, 138.31 (C-2, C-27, C-29), 127.44 (C-3, C-28), 121.01 (C-1), 70.95 (C-14, C-16), 63.18 (C-6), 40.95 (C-24), 32.87 (C-7), 29.80-25.83 (C-8, C-9, C-10, C-11, C-12, C-13, C-17, C-18 C-19, C-20, C-21, C-22).

### N-(11-hydroxyundecyl)-3,5-dinitrobenzamide (DNC11)

White solid. Yield=77%. ¹H NMR (400 MHz, CDCl3): δ 9.04 (m; 1H; H-20), 8.98 (d; J = 2.0 Hz; 2H; H-1, H-18), 3.48 (t; J = 6.7 Hz; 2H; H-3), 3.34 (t; J = 7.3 Hz; 2H; H-13), 1.55 (p; J = 7.4 Hz; 2H; H-12), 1.44 (p; J = 6.9 Hz; 2H; H-4), 1.34 - 1.11 (m; 14H; H-5, H-6, H-7; H-8, H-9, H-10, H11). ¹³C NMR (101 MHz, CDCl3): δ 162.90 (C-16), 148.49, 138.22 (C-17, C-19, C-21), 127.62 (C-1, C-18), 120.67 (C-20), 62.39 (C-3), 40.59 (C-13), 32.43 (C-12), 29.33-25.66 (C-4, C-5, C-6, C-7, C-8, C-9, C-10, C-11).

### Other compounds synthesized using the general methodology described via nucleophilic addition/elimination:

### (3,5-dinitrophenyl)-piperidin-1-yl-methanone (Dnpip)

Replacing the aminoalkanol with piperidine (2.2mmol), K₂CO₃ (2.2mmol) and 3,5-dinitrobenzoyl chloride (1.1mmol) were used in the reaction. White-yellowish solid. Yield=57%. ¹H NMR (400 MHz, CDCl3) δ 9.08 (t, J = 2.1 Hz, 1H), 8.58 (d, J = 2.2 Hz, 2H), 3.76 (s, 2H), 3.35 (s, 2H), 1.74 (s, 4H), 1.59 (s, 2H). ¹³C NMR (101 MHz, CDCI3) δ 165.24, 148.59, 139.91, 127.43, 119.57, 49.11, 43.80, 26.65, 25.49, 24.36.

### (3,5-dinitrophenyl)-4-hydroxypiperidin-1-yl-methanone (DnpipOH)

Replacing the aminoalkanol with 4-hydroxypiperidine (2.2mmol), K₂CO₃ (2.2mmol) and 3,5-dinitrobenzoyl chloride (1.1mmol) were used in the reaction. White-yellowish solid. Yield=52%. ¹H NMR (400 MHz, CDCl3): δ 9.09 (t, J = 2.2 Hz, 1H), 8.59 (d, J = 2.1 Hz, 2H), 4.08 (m, 2H), 3.62 (s, 2H), 3.29 (d, J = 11.6 Hz, 1H), 2.01 (s, 1H), 1.88 (s, 1H), 1.74 (s, 2H), 1.59 (s, 1H). ¹³C NMR (101 MHz, CDCI3) δ 165.38, 148.63, 139.50, 127.44, 119.78, 66.24, 44.98, 39.74, 34.06, 33.54.

### (3,5-dinitrophenyl)-4-benzylpiperidin-1-yl-methanone (DnpipBn)

Replacing the aminoalkanol with 4-benzylpiperidine (2.2mmol), K₂CO₃ (2.2mmol) and 3,5-dinitrobenzoyl chloride (1.1mmol) were used in the reaction. White-yellowish solid. Yield=53%. ¹H NMR (400 MHz, CDCl3): δ 9.08 (t, J = 2.1 Hz, 1H), 8.58 (d, J = 2.1 Hz, 2H), 7.30 (t, J = 7.4 Hz, 2H), 7.22 (d, J = 7.2 Hz, 1H), 7.15 (d, 2H), 4.70 (d, J = 13.3 Hz, 1H), 3.57 (d, J = 13.5 Hz, 1H), 3.11 (t, J = 13.2 Hz, 1H), 2.81 (t, J = 12.9 Hz, 1H), 2.61 (t, J = 6.5 Hz, 2H), 1.91-1.79 (m, J = 12.0, 8.0, 4.0 Hz, 2H), 1.69 (d, J = 13.2 Hz, 1H), 1.36 (d, J = 13.1 Hz, 1H), 1.21 (d, J = 13.4 Hz, 1H). ¹³C NMR (101 MHz, CDCI3) δ 165.25, 148.60, 139.81, 139.56, 129.18, 128.56, 127.45, 126.40, 119.64, 48.40, 43.13, 42.86, 38.22, 32.69, 31.63.

### (3,5-dinitrophenyl)-4-benzylpiperazin-1-yl-methanone (DNpzBn)

Replacing the aminoalkanol with 4-benzylpiperazine (0.9mmol), K₂CO₃ (3mmol) and 3,5-dinitrobenzoyl chloride (0.7mmol) were used in the reaction. White solid. Yield=88%. ¹H NMR (400 MHz, DMSO) δ 8.85 (t, J = 2.2 Hz, 1H), 8.61 (d, J = 2.1 Hz, 2H), 7.38 - 7.19 (m, 6H), 3.66 (t, J = 5.1 Hz, 2H), 3.52 (s, 2H), 3.33 (br s, 2H), 2.47 (d, J = 5.1 Hz, 2H), 2.36 (t, J = 5.2 Hz, 2H). ¹³C NMR (101 MHz, DMSO) δ 164.74, 148.17, 138.87, 137.78, 128.93, 128.26, 127.55, 127.10, 119.15, 61.74, 52.44, 51.77, 47.10, 41.87.

### (3,5-dinitrophenyl)(morpholino)methanone (DNMP)

Replacing the aminoalkanol with morpholine (2.2mmol), K₂CO₃ (2.2mmol) and 3,5-dinitrobenzoyl chloride (1.1mmol) were used in the reaction. White solid. Yield=88%. ¹H NMR (400 MHz, CDCl3): δ 9.09 (t, J = 2.3 Hz, 1H), 8.60 (d, J = 2.2 Hz, 2H), 3.83 (s, 4H), 3.69 (s, 2H), 3.46 (s, 2H). ¹³C NMR (101 MHz, CDCI3) δ 165.38, 148.67, 138.76, 127.67, 119.99, 66.71, 48.35, 43.03.

### 5-(3,5-dinitrobenzamido)pentyl 3,5-dinitrobenzoate (DNC5DN)

Using 5-aminopentan-1-ol (0.4mmol), K₂CO₃ (0.8mmol) and 3,5-dinitrobenzoyl chloride (0.9mmol) were used in the reaction. White solid. Yield=46%. ¹H NMR (400 MHz, DMSO) δ 9.20 (t, J = 5.6 Hz, 1H), 9.05-9.00 (m, J = 2.7, 1.2 Hz, 3H), 8.94 (t, J = 1.8 Hz, 1H), 8.88 (d, J = 2.1 Hz, 2H), 4.42 (t, J = 6.5 Hz, 2H), 3.38 (t, J = 6.3 Hz, 2H), 1.83 (p, J = 6.9 Hz, 2H), 1.66 (p, J = 7.2 Hz, 2H), 1.55 - 1.44 (m, 2H). ¹³C NMR (101 MHz, DMSO) δ 162.67, 162.05, 148.45, 148.26, 137.13, 132.81, 128.87, 127.51, 122.57, 120.82, 66.35, 39.55, 28.44, 27.73, 22.91.

**General Mitsunobu synthetic methodology:** 0.3 mmol of the chosen hydroxyalkyl 3,5-dinitrobenzamide, 2.5 eq. of ADDP and 1 eq of the desired substituted phenol were dissolved in 2 mL of DCM. This solution was then purged with nitrogen, under stirring, for 15 minutes. Then, while maintaining the reactional mixture under nitrogen atmosphere, a solution of 2.5 eq. of triphenylphosphine dissolved in 1 mL of DCM is slowly added to the mixture, over 15 minutes. After the complete addition, the reaction is left stirring at room temperature during 24 hours. Once the reaction is complete, the DCM is evaporated and the residue dissolved in EtOAc. The organic phase is washed with distilled water (2 times), brine, dried with anhydrous Na₂SO₄, and evaporated under reduced pressure. The products were purified by column chromatography.

### Products synthesized via the methodology described:

*N*-(3-(4-methoxyphenoxy)propyl)-3,5-dinitrobenzamide (**DNC30phOMe**) Yield=42%. ¹H NMR (400 MHz, CDCI3) δ 9.09 (t, J = 2.2 Hz, 1H), 8.99 (d, J = 2.1 Hz, 2H), 6.84 - 6.74 (q, J = 6.40, 6.29, 6.29 Hz, 4H), 4.04 (t, J = 5.7 Hz, 2H), 3.71 (s, 3H), 3.64 (t, J = 6.5 Hz, 2H), 2.09 (p, J = 6.1 Hz, 2H). ¹³C NMR (101 MHz, CDCI3) δ 163.30, 154.42, 152.83, 148.90, 138.32, 127.78, 121.24, 115.66, 115.14, 67.43, 56.09, 38.95, 28.93.

*N*-(3-(phenoxy)propyl)-3,5-dinitrobenzamide (**DNC3Oph**) Yield=69%. ¹H NMR (400 MHz, CDCI3) δ 9.16 (t, J = 2.2 Hz, 1H), 8.97 (d, J = 2.1 Hz, 2H), 7.31 (t, J = 7.84 Hz, 2H), 6.99 (d, J = 7.8 Hz, 1H), 6.96 (t, J = 7.8 Hz, 1H), 4.21 (t, J = 5.4 Hz, 2H), 3.78 (q, J = 5.8 Hz, 2H), 2.20 (p, J = 5.5 Hz, 2H). ¹³C NMR (101 MHz, CDCI3) δ 162.49, 158.15, 148.77, 138.05, 129.96, 127.17, 121.66, 121.18, 114.31, 67.55, 40.00, 28.36.

*N*-(3-(4-trifluoromethoxyphenoxy)propyl)-3,5-dinitrobenzamide (**DNC3OPhOCF₃**) Yield=34%. ¹H NMR (400 MHz, CDCI3) δ 9.17 (s, 1H), 8.97 (t, J = 2.4 Hz, 2H), 7.16 (d, J = 8.50 Hz, 2H), 7.12 (d, 2H), 6.94 (dd, J = 9.1, 2.4 Hz, 2H), 4.17 (td, J = 5.5, 2.2 Hz, 2H), 3.78 (d, J = 5.8 Hz, 2H), 2.20 (t, J = 6.3 Hz, 2H). ¹³C NMR (101 MHz, CDCl3) δ 162.58, 156.80, 148.80, 137.95, 127.16, 122.89, 121.25, 115.18, 67.73, 39.55, 28.55.

*N*-(3-(4-fluorophenoxy)propyl)-3,5-dinitrobenzamide (**DNC3OphF**) Yield=70%. ¹H NMR (400 MHz, DMSO) δ 9.05 (d, J = 2.1 Hz, 2H), 8.95 (t, J = 2.2 Hz, 1H), 7.11 (t, J = 8.8 Hz, 2H), 6.94 (dd, J = 9.2, 4.3 Hz, 2H), 4.03 (t, J = 6.1 Hz, 2H), 3.50 (t, J = 6.9 Hz, 2H), 2.01 (p, J = 6.5 Hz, 2H). ¹³C NMR (101 MHz, DMSO) δ 162.22, 157.65, 154.90, 148.22, 137.03, 127.53, 120.85, 115.96, 115.73, 65.76, 36.85, 28.55.

*N*-(3-(4-phenylphenoxy)propyl)-3,5-dinitrobenzamide (**DNC3OphPh**) Yield=62%. ¹H NMR (400 MHz, CDCI3) δ 9.16 (t, J = 1.9 Hz, 1H), 8.99 (d, J = 2.1 Hz, 2H), 7.54 (t, J=1.55 Hz, 4H), 7.42 (t, J = 7.5 Hz, 2H), 7.31 (t, J = 7.3 Hz, 1H), 7.03 (d, J = 8.2 Hz, 2H), 4.25 (t, J = 5.3 Hz, 2H), 3.80 (q, J = 5.8 Hz, 2H), 2.23 (p, J = 5.8 Hz, 2H). ¹³C NMR (101 MHz, CDCI3) δ 162.48, 157.43, 148.78, 140.48, 138.03, 134.75, 128.93, 128.58, 127.18, 127.06, 126.84, 121.20, 114.64, 67.75, 39.98, 28.39.

*N*-(3-(4-trifluoromethylphenoxy)propyl)-3,5-dinitrobenzamide (**DNC3OphCF₃**) Yield=47%. ¹H NMR (300 MHz, CDCl3) δ 9.17 (t, J = 2.1 Hz, 1H), 8.95 (d, J = 2.1 Hz, 2H), 7.57 (d, J = 8.4 Hz, 2H), 7.01 (d, J = 8.5 Hz, 2H), 6.89 (br s, 1H), 4.22 (t, J = 5.5 Hz, 2H), 3.78 (q, J = 6.1 Hz, 2H), 2.22 (p, J = 6.0 Hz, 2H).

*N*-(5-(4-methoxyphenoxy)pentyl)-3,5-dinitrobenzamide (**DNC5OphOMe**) Yield=43%. ¹H NMR (400 MHz, CDCI3) δ 9.14 (s, 1H), 8.94 (t, J = 1.6 Hz, 2H), 6.80 (s, 4H), 6.63 (br s, 1H), 3.93 (t, *J* = 6.1 Hz, 2H), 3.75 (s, 3H), 3.57 (q, J = 6.6 Hz, 2H), 1.83 (p, J = 6.6 Hz, 2H), 1.75 (q, J = 7.4 Hz, 2H), 1.61 (q, J = 7.8 Hz, 2H). ¹³C NMR: (101 MHz, CDCl3) δ 162.89, 153.91, 148.72, 138.19, 127.27, 121.12, 115.48, 114.76, 68.33, 40.82, 29.16, 29.01, 23.75.

*N*-(5-(phenoxy)pentyl)-3,5-dinitrobenzamide (**DNC50ph**) Yield=92%. ¹H NMR (400 MHz, DMSO) δ 9.20 (t, J = 5.7 Hz, 1H), 9.05 (d, J = 2.1 Hz, 2H), 8.94 (t, J = 2.0 Hz, 1H), 7.25 (t, J = 7.8 Hz, 2H), 6.90 (d, J = 7.5 Hz, 3H), 3.95 (t, J = 6.4 Hz, 2H), 3.37 (t, J = 6.4 Hz, 2H), 1.75 (p, J = 6.8 Hz, 2H), 1.64 (p, J = 7.1 Hz, 2H), 1.54-1.43 (m, 2H). ¹³C NMR (101 MHz, DMSO) δ 161.97, 158.64, 148.21, 137.09, 129.47, 127.47, 120.77, 120.37, 114.37, 67.12, 39.68, 28.55, 28.43, 23.09.

*N*-(5-(4-trifluoromethoxyphenoxy)pentyl)-3,5-dinitrobenzamide (**DNC5OPhOCF₃**) Yield=84%. ¹H NMR (400 MHz, CDCI3) δ 9.16 (t, J = 3.0 Hz, 1H), 8.95 (d, J = 2.1 Hz, 2H), 7.12 (d, J = 8.5 Hz, 2H), 6.85 (d, J = 2.1 Hz, 2H), 6.56 (br s, 1H), 3.97 (t, J = 6.1 Hz, 2H), 3.58 (q, J = 6.7 Hz, 2H), 1.86 (p, J = 7.4 Hz, 2H), 1.77 (p, J = 7.5 Hz, 2H), 1.59 (p, J = 7.1 Hz, 2H). ¹³C NMR (101 MHz, CDCI3) δ 162.89, 157.54, 148.76, 138.15, 127.25, 122.60, 121.19, 115.23, 68.08, 40.80, 29.31, 28.92, 23.70.

*N*-(5-(4-fluorophenoxy)pentyl)-3,5-dinitrobenzamide (**DNC50phF**) Yield=87%. ¹H NMR (400 MHz, CDCI3) δ 9.13 (t, J = 2.3 Hz, 1H), 8.95 (d, J = 2.2 Hz, 2H), 6.94 (t, J = 8.6 Hz, 2H), 6.79 (dd, J = 9.1, 4.3 Hz, 2H), 6.71 (t, J = 5.8 Hz, 1H), 3.93 (t, J = 6.1 Hz, 2H), 3.57 (q, J = 6.7 Hz, 2H), 1.84 (p, J = 7.0, 6.5, 6.5 Hz, 2H), 1.76 (p, *J* = 7.2 Hz, 2H), 1.60 (p, J = 15.0, 10.3, 6.0 Hz, 2H). ¹³C NMR (101 MHz, CDCI3) δ 162.91, 158.46, 156.10, 155.08, 148.72, 138.14, 127.28, 121.15, 116.03, 115.80, 115.47, 115.40, 68.26, 40.81, 29.25, 28.97, 23.71.

*N*-(5-(4-chlorophenoxy)pentyl)-3,5-dinitrobenzamide (**DNC50phCl**) Yield=75%. ¹H NMR (400 MHz, CDCI3) δ 9.14 (t, J = 2.1 Hz, 1H), 8.95 (d, J = 2.1 Hz, 2H), 7.19 (d, *J* = 2.1 Hz, 2H), 6.79 (d, J = 8.9 Hz, 2H), 6.64 (t, J = 5.9 Hz, 1H), 3.94 (t, J = 6.2 Hz, 2H), 3.57 (q, J = 6.7 Hz, 2H), 1.85 (p, *J* = 7.0, 6.0 Hz, 2H), 1.76 (p, J = 7.4 Hz, 2H), 1.60 (p, *J* = 7.4, 7.0 Hz, 2H). ¹³C NMR (101 MHz, CDCI3) δ 162.89, 157.60, 148.73, 138.13, 129.44, 127.26, 125.64, 121.16, 115.77, 67.93, 40.79, 29.27, 28.89, 23.68.

*N*-(5-(4-bromophenoxy)pentyl)-3,5-dinitrobenzamide (**DNC5OphBr**) ¹H NMR (400 MHz, DMSO) δ 9.20 (t, J = 5.6 Hz, 1H), 9.05 (d, J = 2.1, 0.9 Hz, 2H), 8.95 (q, J = 1.8 Hz, 1H), 7.40 (d, *J* = 8.3 Hz, 2H), 6.88 (d, *J* = 8.3 Hz, 2H), 3.95 (t, J = 6.4 Hz, 2H), 3.4-3.31 (m, 2H), 1.74 (p, J = 6.7 Hz, 2H), 1.63 (p, J = 7.2 Hz, 2H), 1.47 (p, *J* = 7.4, 6.6 Hz, 2H). ¹³C NMR (101 MHz, DMSO) δ 161.97, 157.94, 148.20, 137.08, 132.10, 127.46, 120.77, 116.71, 111.74, 67.62, 39.61, 28.50, 28.27, 22.99.

*N*-(5-(4-phenylphenoxy)pentyl)-3,5-dinitrobenzamide (**DNC50phPh**) Yield=66%. ¹H NMR (400 MHz, CDCl3) δ 9.16 (d, J = 2.2 Hz, 1H), 8.95 (d, J = 2.2 Hz, 2H), 7.52 (dd, J = 11.8, 8.1 Hz, 4H), 7.41 (t, J = 7.1 Hz, 2H), 7.31 (t, J = 7.7 Hz, 1H), 6.95 (d, J = 8.1 Hz, 2H), 6.46 (br s, 1H), 4.04 (t, J = 6.0 Hz, 2H), 3.60 (q, J = 6.7 Hz, 2H), 1.90 (p, J = 7.2 Hz, 2H), 1.79 (p, J = 7.6 Hz, 2H), 1.71-1.51 (m, 2H).

*N*-(5-(4-benzylphenoxy)pentyl)-3,5-dinitrobenzamide (**DNC50phBn**) Yield=88%. ¹H NMR (400 MHz, DMSO) δ 9.20 (t, J = 5.7 Hz, 1H), 9.05 (d, J = 2.1 Hz, 2H), 8.94 (t, J = 2.1 Hz, 1H), 7.29-7.12 (m, 5H), 7.10 (d, J = 8.3 Hz, 2H), 6.82 (d, J = 8.3 Hz, 2H), 3.91 (t, J = 6.4 Hz, 2H), 3.84 (s, 2H), 3.39-3.30 (m, 2H), 1.73 (p, J = 6.8 Hz, 2H), 1.62 (p, J = 7.1 Hz, 2H), 1.46 (p, J = 8.4, 7.1 Hz, 2H). ¹³C NMR (101 MHz, DMSO) δ 161.96, 156.96, 148.19, 141.79, 137.08, 133.13, 128.59, 128.58, 127.46, 125.85, 120.75, 114.35, 67.22, 40.23, 39.98, 39.78, 39.64, 39.57, 28.54, 28.44, 23.08.

*N*-(5-(4-trifluoromethylphenoxy)pentyl)-3,5-dinitrobenzamide (**DNC5OphCF₃**) Yield=71%. ¹H NMR (400 MHz, CDCI3) δ 9.07 (q, J = 3.2, 2.6 Hz, 1H), 9.01 (t, J = 2.7 Hz, 2H), 7.45 (d, J = 8.4 Hz, 2H), 6.88 (d, J = 8.4 Hz, 2H), 3.97 (t, J = 6.1 Hz, 2H), 3.44 (dd, J = 8.3, 5.6 Hz, 2H), 1.81 (p, J = 6.9, 6.3 Hz, 2H), 1.68 (q, J = 7.5 Hz, 2H), 1.53 (p, *J* = 7.3, 6.7 Hz, 2H). ¹³C NMR (101 MHz, CDCI3) δ 163.20, 161.40, 148.54, 138.15, 127.61, 126.85, 120.81, 114.38, 113.17, 67.90, 40.39, 28.97, 28.74, 23.50.

*N*-(5-(4-nitrophenoxy)pentyl)-3,5-dinitrobenzamide (**DNC5OphNO₂**) Yield=32%. ¹H NMR (400 MHz, DMSO) δ 9.21 (t, J = 5.4 Hz, 1H), 9.04 (d, J = 2.2 Hz, 2H), 8.94 (t, J = 2.1 Hz, 1H), 8.18 (d, J = 8.9 Hz, 2H), 7.12 (d, J = 8.9 Hz, 2H), 4.13 (t, J = 6.4 Hz, 2H), 3.4-3.3 (m, 2H), 1.80 (p, J = 6.7 Hz, 2H), 1.64 (p, J = 7.1 Hz, 2H), 1.49 (p, J = 7.9, 7.4 Hz, 2H).¹³C NMR (101 MHz, DMSO) δ 164.05, 162.00, 148.22, 140.69, 137.07, 127.47, 125.93, 120.80, 115.00, 68.53, 39.58, 28.46, 28.09, 22.88.

*N*-(9-(4-methoxyphenoxy)nonyl)-3,5-dinitrobenzamide (**DNC9OphOMe**) Yield=36%. ¹H NMR (400 MHz, CDCI3) δ 9.15 (q, J = 1.8 Hz, 1H), 8.95 (t, J = 1.5 Hz, 2H), 6.81 (d, J = 1.1 Hz, 4H), 6.51 (s, 1H), 3.89 (t, J = 6.5 Hz, 2H), 3.76 (d, J = 1.1 Hz, 3H), 3.52 (q, J = 6.7 Hz, 2H), 1.74 (p, J = 6.8 Hz, 2H), 1.67 (p, *J* = 7.0 Hz, 2H), 1.50-1.30 (m, 10H). ¹³C NMR (101 MHz, CDCI3) δ 162.83, 153.74, 153.33, 148.73, 138.28, 127.27, 121.12, 115.50, 114.71, 68.68, 55.87, 40.95, 29.55, 29.51, 29.45, 29.39, 29.27, 27.04, 26.12.

*N*-(9-(phenoxy)nonyl)-3,5-dinitrobenzamide (**DNC90ph**) Yield=93%. ¹H NMR (400 MHz, CDCI3) δ 9.17 (d, J = 2.3 Hz, 1H), 8.98 (d, J = 2.1 Hz, 2H), 7.29 (t, J = 7.8 Hz, 2H), 7.00 - 6.84 (m, 3H), 6.61 (br s, 1H), 3.96 (t, J = 6.5 Hz, 2H), 3.54 (q, J = 6.8 Hz, 2H), 1.79 (p, J = 6.7 Hz, 2H), 1.69 (p, J = 7.0 Hz, 2H), 1.53 - 1.30 (m, 10H).

*N*-(11-(4-methoxyphenoxy)undecyl)-3,5-dinitrobenzamide (**DNC11OphOMe**) Yield=85%. ¹H NMR (400 MHz, CDCI3) δ 9.14 (t, J = 1.8 Hz, 1H), 8.94 (d, J = 2.1 Hz, 2H), 6.81 (s, 4H), 6.55 (t, J = 5.7 Hz, 1H), 3.89 (t, J = 6.5 Hz, 2H), 3.76 (s, 3H), 3.52 (q, J = 6.8 Hz, 2H), 1.74 (p, J = 7.0 Hz, 2H), 1.66 (p, J = 5.6, 4.0 Hz, 2H), 1.49-1.26 (m, 14H). ¹³C NMR (101 MHz, CDCI3) δ 162.85, 153.73, 153.36, 148.73, 138.28, 127.26, 121.10, 115.52, 114.72, 68.76, 55.87, 40.98, 29.62, 29.52, 29.47, 29.36, 27.08, 26.15.

*N*-(11-(phenoxy)undecyl)-3,5-dinitrobenzamide (**DNC11Oph**) Yield=70%. ¹H NMR (400 MHz, DMSO) δ 9.18 (t, J = 5.5 Hz, 1H), 9.05 (d, J = 2.0 Hz, 2H), 8.94 (t, J = 2.2 Hz, 1H), 7.26 (t, J = 7.8 Hz, 2H), 6.94 - 6.85 (m, 3H), 3.92 (t, J = 6.5 Hz, 2H), 3.32 (q, J = 6.7 Hz, 2H), 1.68 (p, J = 6.7 Hz, 2H), 1.55 (t, J = 6.9 Hz, 2H), 1.44 - 1.23 (m, 14H). ¹³C NMR (101 MHz, DMSO) δ 161.94, 158.67, 148.22, 137.11, 129.49, 127.49, 120.78, 120.36, 114.37, 67.22, 29.06, 29.01, 28.84, 28.79, 28.74, 26.50, 25.58.

(3,5-dinitrophenyl)(4-(4-methoxyphenoxy)piperidin-1-yl)methanone (**DnpipOPhOMe**) Yield=29%. ¹H NMR ¹³C NMR:
2-(3,5-dinitrophenyl)-4,5-dihydrooxazole **(DNC2cycle):** Yield=70%. ¹H NMR (400 MHz, DMSO-D6): δ 8.88 (m; 1H; H-10), 8.75 (m; 2H; H-1, H-8), 4.49 (t; J = 9.6 Hz; 2H; H-4), 4.01 (t; J = 9.6 Hz; 2H; H-3). ¹³C NMR (101 MHz, DMSO-D6): δ 160.19 (C-6), 148.41, 130.18 (C-11, C-9, C-7), 127.41 (C-1, C-8), 120.95 (C-10), 68.83 (C-4), 54.86 (C-3).

2-(3,5-dinitrophenyl)-5,6-dihydro-4H-1,3-oxazine (**DNC3cycle**): Yield=59%. ¹H NMR (400 MHz, DMSO-D6): δ 8.89 (t; J = 2.2 Hz; 1H; H-2), 8.83 (d; J = 2.2 Hz; 2H; H-4, H-12), 4.45 (t; J = 5.4 Hz; 2H; H-8), 3.59 (t; J = 5.8 Hz; 2H; H-10), 1.95 (p; J = 5.6 Hz; 2H; H-9). ¹³C NMR (101 MHz, DMSO): δ 150.99 (C-6), 148.21, 136.61 (C-1, C-3, C-5), 126.14 (C-4, C-12), 119.93 (C-2), 65.79 (C-8), 42.23 (C-10), 21.18 (C-9).

### Synthesis of DNC5pip

1eq of *N*-(5-hydroxypentyl)-3,5-dinitrobenzamide was dissolved in 1mL of pyridine and stirred at 0°C. Then, 1.2eq of methanesulfonyl chloride were added to the reaction. After 2h, the mixture was dissolved in EtOAc and washed with distilled water and brine, and the organic phase was dried with anhydrous Na₂SO₄, filtered, and concentrated under reduced pressure. After this, the product was used without further purification. 1eq of this product (0.17mmol) was dissolved in 1mL of DMF. To this, 1eq (0.26mmol) of piperidine and 2eq (0.34mmol) of NaH, dissolved in 0.5 mL of DMF, were added. At the endpoint, the reaction was washed with distilled water and brine. Then, the organic phase was dried with anhydrous Na₂SO₄, filtered, and concentrated under reduced pressure. After this, the product was purified by column chromatography. Brown solid. Yield=40%. ¹H NMR (300 MHz, CDCI3) δ 9.26 (d, J = 2.1 Hz, 2H), 9.09 (t, J = 2.1 Hz, 1H), 8.76 (br s, 1H), 3.55 (q, J = 6.0 Hz, 2H), 3.08 (br s, 4H), 2.97 (t, J = 7.2 Hz, 2H), 2.01 (t, J = 5.6 Hz, 4H), 1.92 (t, J = 7.3 Hz, 2H), 1.77 (q, J = 6.8 Hz, 2H), 1.71 -1.53 (m, 4H).

### Synthesis of DN(NC5N)DN

1mmol of *N*-(5-hydroxypentyl)-3,5-dinitrobenzamide was dissolved in toluene/SOCl₂ (1/1 in volume, total volume 2mL). Then, 0,1 mL of DMF were added to the reaction mixture, and the mixture was heated to 80°C. After 16h, the solvent was evaporated, and the product was purified by column chromatography yielding *N*-(5-chloropentyl)-3,5-dinitrobenzamide.
Then, the previous product, *N*-(5-chloropentyl)-3,5-dinitrobenzamide (0.97 mmol), 0.3 mmol of NaI and 3eq of NaN₃ were dissolved in 10mL of ACN. The reaction was heated to 70°C and stirred for 5 days. Afterwards, the ACN was removed under reduced pressure, the residue was redissolved in EtOAc and washed with distilled water and brine. Then the organic phase was dried with anhydrous Na₂SO₄, concentrated under reduced pressure and purified by column chromatography, yielding *N*-(5-azidopentyl)-3,5-dinitrobenzamide.

After, 0.94 mmol of *N*-(5-azidopentyl)-3,5-dinitrobenzamide was dissolved in 6mL of THF. To this, 1.9mmol of PPh₃ were added and the reaction was placed under N₂ atmosphere and stirred for 24h. Afterwards, 0.1mL of water were added, and the reaction was stirred for another 24h. Finally, the solvent was evaporated, and the mixture was used without further purification yielding *N*-(5-aminopentyl)-3,5-dinitrobenzamide.

The *N*-(5-aminopentyl)-3,5-dinitrobenzamide was dissolved in 30mL of EtOAc. To this, 1.9mmol of K₂CO₃ were added and the reaction was stirred for 15 min. Then, 0.94mmol of 3,5-dinitro benzoyl chloride, dissolved in 5mL of EtOAc, were added dropwise to the mixture. When completed the reaction was washed with distilled water and brine, the organic phase was dried with anhydrous Na₂SO₄, filtered, and concentrated under reduced pressure. After this, the product was purified by column chromatography. White solid. ¹H NMR (400 MHz, DMSO-D6): δ 9.19 (t; J = 5.6 Hz; 2H; H-5, H-11), 9.03 (d; J = 2.0 Hz; 4H; H-2, H-14, H-18, H-21), 8.94 (t; J = 2.1 Hz; 2H; H-16, H-23), 3.33 (d; J = 5.9 Hz; 4H; H-6, H-10), 1.62 (p; J = 7.2 Hz; 4H; H-7, H-9), 1.41 (d; J = 7.2 Hz; 2H; H-8). ¹³C NMR (101 MHz, DMSO-D6) δ 161.98 (C-4, C12), 148.19, 137.10 (C-1, C-3, C-13, C-15, C-17, C-22), 127.47 (C-2, C-21, C-14, C-18), 120.75 (C-16, C-23), 39.61 (C-6, C-10), 28.49 (C-7, C-9), 23.89 (C-8).

### Synthesis of DnpzMe₂

4-(3,5-dinitrobenzoyl)-1,1-dimethylpiperazin-1-ium (**DnpzMe₂**) (3,5-dinitrophenyl)-piperazin-1-yl-methanone and 2.8mmol of K₂CO₃ were dissolved in 5mL of ACN. Then 2mmol of CH₃I were added to the mixture and the reaction was heated up to 60°C. After 2h, the reaction was stopped, the solid was filtered off and the ACN was evaporated under reduced pressure. Then, the solid was recrystalized in acetone, yielding the final product. Orange solid. ¹H NMR (400 MHz, DMSO) δ 8.91 (t, J = 2.5 Hz, 1H), 8.65 (s, 2H), 4.00 (s, 2H), 3.69 (s, 2H), 3.55 (s, 2H), 3.44 (s, 2H), 3.19 (s, 6H). ¹³C NMR (101 MHz, DMSO) δ 165.18, 148.32, 137.59, 127.67, 119.60, 59.71, 50.66, 36.10.

**General synthetic methodology for C2 derivatives:** 1 mmol of ethanolamine is stirred with di-tert-butyl carbonate (2 eq.), at room temperature, for 30 minutes, yielding the desired product tert-butyl (2-hydroxyethyl)carbamate. Then, this product is dissolved in pyridine and methanesulfonyl chloride (1.1 eq.) is added. The mixture stirred at room temperature for 1 hour. The reactional mixture is then dissolved in EtOAc and is washed with a solution of HCl (1M) 2 times, then with a saturated aqueous solution of sodium hydrogen carbonate, distilled water, brine, dried with anhydrous Na₂SO₄, and evaporated under reduced pressure. The product 2-((tert-butoxycarbonyl)amino)ethyl methanesulfonate was used without further purification.

The previous product was dissolved in DCM and added to a mixture of the desired substituted phenol (1.5 eq.) dissolved in a aqueous solution of potassium hydroxide (50% m/v). Then, tetrabutylammonium chloride (0.1 eq.) was added and the mixture vigorously stirred at room temperature overnight. Upon completion, additional distilled water and DCM were added and the phases separated. The organic phase was washed with distilled water, brine, dried with anhydrous Na₂SO₄, and evaporated under reduced pressure. The products were purified by column chromatography.

The obtained intermediates were then dissolved in 3 mL of a mixture of DCM and trifluoroacetic acid (1:1) and stirred at room temperature for 1 hour. Then, ethyl acetate and a large excess of N,N-diisopropylethylamine were added and the mixture stirred at room temperature for an additional 1 hour. Finally, 3,5-dinitro benzoyl chloride (1 eq.) or 3-nitro-5-(trifluoromethyl)benzoyl chloride (1 eq.) was added to the previous mixture and stirred at room temperature for 1 hour. Then, additional ethyl acetate was added and the organic phase was washed with distilled water (3 times), brine, dried with anhydrous Na₂SO₄, and evaporated under reduced pressure. The final products were purified by column chromatography.

### Biological assays

### Bacterial strains and culture conditions

*Mycobacterium tuberculosis* H37Rv (ATCC 27294) were cultivated in Middlebrook 7H9 medium supplemented with OADC and 0.05 % tyloxapol (Sigma-Aldrich) and incubated at 37 ºC until exponential growth phase was achieved. Determination of the Minimum Inhibitory Concentration (MIC) and Minimum Bactericidal Concentrations (MBC)

The MICs were determined by the broth microdilution method in 96-well plates. Briefly, M. tuberculosis bacterial cultures in exponential growth phase were collected by centrifugation, washed in PBS and re-suspended in fresh culture medium. Clumps of bacteria were removed by ultrasonic treatment of the bacteria suspension in an ultrasonic water bath for 5 min followed by a low-speed centrifugation (500 × g) for 2 min. Single cell suspension was verified by microscopy. The microplates containing a bacterial suspension corresponding to approximately 105 colony-forming units per ml were incubated with the selected concentrations of the compounds. Every other day, the optical density of the wells was measured in a Tecan M200 spectrophotometer, following 30 s of orbital agitation. These values were used to produce the growth curves. At the 10^{th} day of incubation, the MIC was determined, corresponding to the concentration with no visible turbidity. Optical density measures were taken until the 15^{th} day of incubation following which the bacterial samples were recovered from the MIC test microplates and plated in 7H10 + OADC solid medium. The MBC was determined following 3 weeks of incubation, corresponding to the concentration of compound that produced no colonies on the solid medium. Bacteria treated with DMSO solvent at the same proportions as present during the compound tests were used as a control. Isoniazid was used as a positive control for bacteria killing and assay validation following EUCAST guidelines.

### Liposomal formulation

Liposomes were obtained by hydration of a solution containing the lipids and the compound to be incorporated, following the following steps:

The lipids used were dimyristoylphosphatidylcholine (DMPC) and dimyristoylphosphatidylglycerol (DMPG). The lipids and the prodrug were weighed, transferred to a round bottom flask, dissolved in dichloromethane and the organic solvent was evaporated at a rotary evaporator to form a lipid film. The flask was dried in a vacuum pump to remove any remaining solvent and 1 ml of isotonic phosphate buffer pH 7.4 (PBS) was added at a temperature at least 10°C higher than the phase transition temperature of the lipids present. The flask was then stirred for five minutes in order to hydrate the lipid film completely, waited a few minutes and stirred again for another five minutes after which the mixture was placed in an ultrasonic bath for 3 periods of 2 minutes, interspersed with 2 minutes rest. All prepared suspensions were observed under a phase-contrast optical microscope at 400x magnification.

To determine the incorporation efficiency (EE), an aliquot of the liposomal suspension obtained after hydration was taken and another aliquot of the liposomal suspension obtained after centrifugation, removal of the supernatant, and resuspension of the pellet in PBS in the original volume it had before centrifugation. The EEs were then calculated as the ratio of the concentration of the compound in the resuspended liposomal suspension/concentration of the compound in the initial liposomal suspension.

The term "comprising" whenever used in this document is intended to indicate the presence of stated features, integers, steps, components, but not to preclude the presence or addition of one or more other features, integers, steps, components or groups thereof.

The disclosure should not be seen in any way restricted to the embodiments described and a person with ordinary skill in the art will foresee many possibilities to modifications thereof. The above described embodiments are combinable.

Where ranges are provided, the range limits are included. Furthermore, it should be understood that unless otherwise indicated or otherwise evident from the context and/or understanding of a technical expert, the values which are expressed as ranges may assume any specific value within the ranges indicated in different achievements of the invention, at one tenth of the lower limit of the interval, unless the context clearly indicates the contrary. It should also be understood that, unless otherwise indicated or otherwise evident from the context and/or understanding of a technical expert, values expressed as range may assume any sub-range within the given range, where the limits of the sub-range are expressed with the same degree of precision as the tenth of the unit of the lower limit of the range.

The dependent claims further set out particular embodiments of the disclosure.

### References

1. World Health Organization Global Tuberculosis Report; 2021; ISBN 9789240037021.
2. Chikhale, R. V.; Barmade, M.A.; Murumkar, P.R.; Yadav, M.R. Overview of the Development of DprE1 Inhibitors for Combating the Menace of Tuberculosis. J. Med. Chem. 2018, 61, 8563-8593, doi:10.1021/acs.jmedchem.8b00281.
3. Imran, M.; A.S., A.; Thabet, H.K.; Abida; Afroz Bakht, M. Synthetic Molecules as DprE1 Inhibitors: A Patent Review. Expert Opin. Ther. Pat. 2021, 31, 759-772, doi:10.1080/13543776.2021.1902990.
4. Degiacomi, G.; Belardinelli, J.M.; Pasca, M.R.; Rossi, E. De; Riccardi, G.; Chiarelli, L.R. Promiscuous Targets for Antitubercular Drug Discovery: The Paradigm of DprE1 and MmpL3. Appl. Sci. 2020, 10, 1-19, doi:10.3390/appl0020623.
5. Vadim A. Makarov, Stewart T. Cole, U.M. New Benzothiazinone Derivatives and Their Use as Antibacterial Agents. WO2007134625A1 2007.
6. Makarov, V.; Manina, G.; Mikusova, K.; Möllmann, U.; Ryabova, O.; Saint-Joanis, B.; Dhar, N.; Pasca, M.R.; Buroni, S.; Lucarelli, A.P.; et al. Benzothiazinones Kill Mycobacterium Tuberculosis by Blocking Arabinan Synthesis. Science (80-.). 2009, 324, 801-804, doi:10.1126/science.1171583.
7. Christophe, T.; Jackson, M.; Hee, K.J.; Fenistein, D.; Contreras-Dominguez, M.; Kim, J.; Genovesio, A.; Carralot, J.P.; Ewann, F.; Kim, E.H.; et al. High Content Screening Identifies Decaprenyl-Phosphoribose 2' Epimerase as a Target for Intracellular Antimycobacterial Inhibitors. PLoS Pathog. 2009, 5, doi:10.1371/journal.ppat.1000645.
8. Richter, A.; Rudolph, I.; Möllmann, U.; Voigt, K.; Chung, C. wa; Singh, O.M.P.; Rees, M.; Mendoza-Losana, A.; Bates, R.; Ballell, L.; et al. Novel Insight into the Reaction of Nitro, Nitroso and Hydroxylamino Benzothiazinones and of Benzoxacinones with Mycobacterium Tuberculosis DprE1. Sci. Rep. 2018, 8, 1-12, doi:10.1038/s41598-018-31316-6.
9. Verma, H.; Choudhary, S.; Singh, P.K.; Kashyap, A.; Silakari, O. Decoding the Signature of Molecular Mechanism Involved in Mutation Associated Resistance to 1, 3-Benzothiazin-4-Ones (Btzs) Based DprE1 Inhibitors Using BTZ043 as a Reference Drug. Mol. Simul. 2019, 45, 1515-1523, doi:10.1080/08927022.2019.1659507.
10. Tiwari, R.; Möllmann, U.; Cho, S.; Franzblau, S.G.; Miller, P.A.; Miller, M.J. Design and Syntheses of Anti-Tuberculosis Agents Inspired by BTZ043 Using a Scaffold Simplification Strategy. ACS Med. Chem. Lett. 2014, 5, 587-591, doi:10.1021/mI500039g.
11. Wang, A.; Huang, G.; Wang, B.; Lv, K.; Wang, H.; Tao, Z.; Liu, M.; Guo, H.; Lu, Y. Design, Synthesis and Antimycobacterial Activity of 3,5-Dinitrobenzamide Derivatives Containing Fused Ring Moieties. Bioorganic Med. Chem. Lett. 2018, 28, 2945-2948, doi:10.1016/j.bmcl.2018.07.005.
12. Munagala, G.; Yempalla, K.R.; Aithagani, S.K.; Kalia, N.P.; Ali, F.; Ali, I.; Rajput, V.S.; Rani, C.; Chib, R.; Mehra, R.; et al. Synthesis and Biological Evaluation of Substituted N-Alkylphenyl-3,5-Dinitrobenzamide Analogs as Anti-TB Agents. Medchemcomm 2014, 5, 521-527, doi:10.1039/c3md00366c.

## Claims

1. Compound of formula I or a pharmaceutically acceptable salt, or ester or solvate thereof 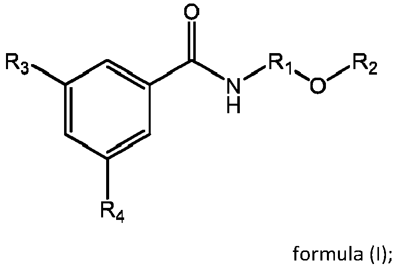
wherein R₁, R₂, R₃ and R₄ are independently selected from each other;
R₁ is an alkyl chain, an alkenyl chain or an alkynyl chain with at least 2 carbons;
R₂ is selected from H or a substituted or unsubstituted aryl;
R₃ and R₄ are selected from NO₂ and CF₃;
provided that if R₁ is a C₂ chain then R₂ is not: a fluor-substituted phenyl, a chloro-substituted phenyl, a phenyl, a methoxyphenyl, a hydroxyphenyl, a trifluoromethoxyphenyl, a benzoate, or an amino-phenyl;
provided that if R₁ is a C₃ chain then R₂ is not 4-methoxyphenyl.

2. Compound according to the previous claim wherein R₁ is an unsubstituted alkyl chain.

3. Compound according to any of the previous claims wherein R₃ and R₄ are NO₂ or R₃ is NO₂ and R₄ is CF₃.

4. Compound according to any of the previous claims provided that if R₃ and R₄ are NO₂ and R₁ is a C₂ chain then R₂ is selected from: H; biphenyl; 4-bromophenyl; 4-benzylphenyl; 4-trifluoromethylphenyl.

5. Compound according to any of the previous claims wherein R₁ is an alkyl chain, an alkenyl chain or an alkynyl chain with at least 3 carbons; preferably an alkyl chain with at least 3 carbons.

6. Compound according to any of the previous claims wherein R₁ is an alkyl chain, an alkenyl chain or an alkynyl chain with at least 4 carbons; preferably an alkyl chain with at least 4 carbons.

7. Compound according to any of the previous claims wherein R₁ chain ranges from C₃ - C₃₀; preferably C₄-C₃₀; more preferably C₅-C₃₀.

8. Compound according to any of the previous claims wherein R₂ is a 4-substituted phenyl selected from: 4-fluorophenyl; 4-biphenyl; 4-(trifluoromethyl)phenyl; 4-(trifluoromethoxy)phenyl; 4-nitrophenyl; 4-clorophenyl;4-benzylphenyl; 4-bromophenyl; 4-methoxyphenyl; 4-benzylphenyl; 4-biphenyl.

9. Compound according to any of the previous claims wherein R₁ is a C₃ chain and R₂ is selected from: biphenyl; 4-(trifluoromethoxy)phenyl; 4-fluorophenyl; 4-(trifluoromethyl)phenyl; phenyl; or R₁ is a C₅ chain and R₂ is selected from: biphenyl; 4-methoxyphenyl; 4-(trifluoromethoxy)phenyl; 4-nitrophenyl; 4-fluorophenyl; 4-clorophenyl; 4-(trifluoromethyl)phenyl; 4-benzylphenyl; 4-bromophenyl; phenyl.

10. Compound according to any of the previous claims wherein R₁ is a C₉ chain and R₂ is 4-methoxyphenyl or phenyl; or R₁ is a C₁₁ chain and R₂ is 4-methoxyphenyl or phenyl; or R₁ is a C₉ chain and R₂ is (C₈H₁₆)CH₂OH or H.

11. Compound according to any of the previous claims wherein R₂ is H and R₁ is selected from: C₅ chain; C₃ chain; C₂ chain; and C₁₁ chain.

12. Compound according to any of the previous claims wherein the compound is

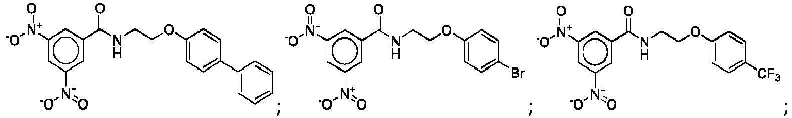

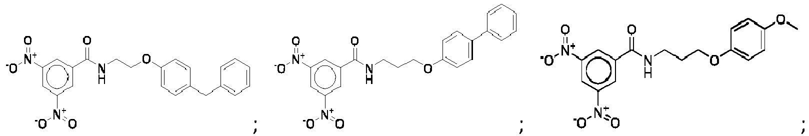

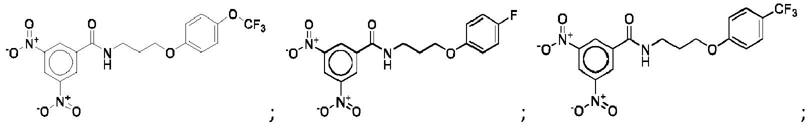

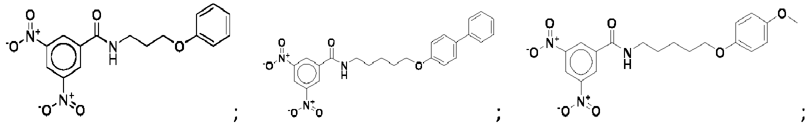

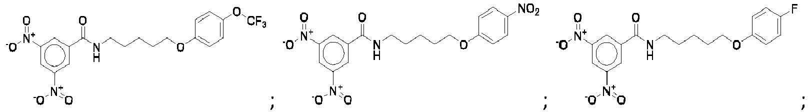

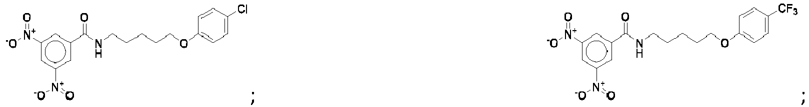

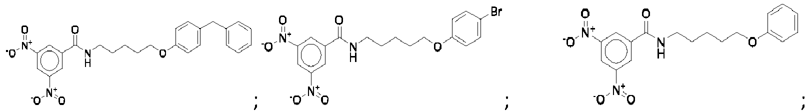

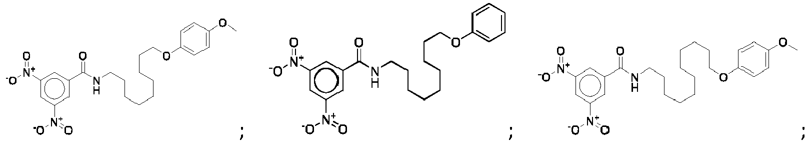

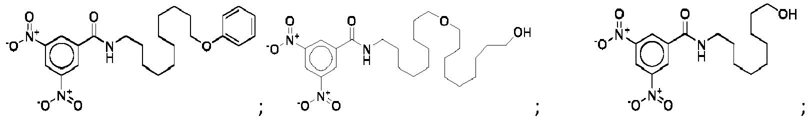

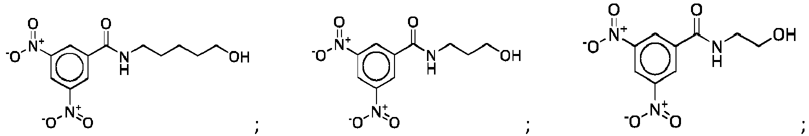

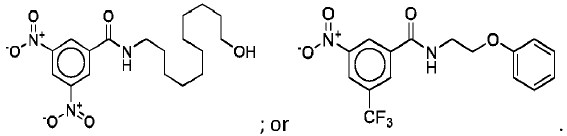

13. Compound according to any of the previous claims for use in medicine or veterinary, preferably for use in any condition susceptible of being improved or prevented by selective inhibition of the enzyme DprE1.

14. Compound according to the previous claim for use in the treatment or prevention of a mycobacterial infection.

15. Compound according to any of the previous claims 13-14 for use in the treatment or prevention of tuberculosis; preferably the tuberculosis is caused by an organism from the *Mycobacterium Tuberculosis* Complex; preferably *Mycobacterium bovis* or *Mycobacterium tuberculosis.*
